# EUROPEAN PATENT APPLICATION

(11) **EP 4 663 168 A1**
(43) Date of publication of application: **17.12.2025**
(21) Application number: 25179434.3
(22) Date of filing: 28.05.2025
(51) Int. Cl.: A61F 2/82, A61F 2/848

(54) **STENT DEVICE WITH EXTENSION FOR TREATING BLADDER NECK**

(30) Priority: 13.06.2024 US 202463659473 P
(71) Applicant: Medi-Tate Ltd., 3850169 Hadera (IL)
(72) Inventor: Kilemnik, Ido, 4286500 Haniel (IL)
(74) Representative: Hoener, Matthias

(57) **Abstract**

It is an object of the disclosed technique to provide methods and systems for a stent device, such as a prostatic urethral implant, for treating prostate enlargement and a constriction of the prostatic urethra while also maintaining the bladder neck and internal urethral sphincter unobstructed.

## Description

### FIELD OF THE DISCLOSED TECHNIQUE

The disclosed technique relates to systems and methods for relieving prostate enlargement (e.g., as a result of benign prostatic hyperplasia), in general, and to systems and methods for enlarging the prostatic urethra and unobstructing the internal urethral sphincter and the bladder neck.

### BACKGROUND OF THE DISCLOSED TECHNIQUE

The prostate is a walnut-sized gland that forms part of the male reproductive system. The prostate is located in front of the rectum and just below the bladder, where urine is stored. The urethra is the canal connecting the bladder to the penis through which urine can be passed out of the body and can be sub-divided into four different anatomical sections, known as the pre-prostatic urethra, the prostatic urethra, the membranous urethra and the spongy urethra. The pre-prostatic urethra passes through the bladder neck, which is the distal section of the bladder proximate to the prostate. In the male reproductive system, the ejaculatory ducts are the tubes connecting the testes to the urethra through which semen can also be passed out of the body. The prostate surrounds a portion of the urethra, which is referred to as the prostatic urethra, with the ejaculatory ducts also passing through the prostate and connecting with the prostatic urethra. A short section of the urethra distal to the prostatic urethra is the membranous urethra which sits at the bulb of the penis. The spongy urethra is the portion of the urethra that runs along the length of the penis, ending with the urethral meatus. Two sphincter muscles control the release of urine from the bladder. The internal urethral sphincter, located in the bladder neck at the intersection between the pre-prostatic urethra and the prostatic urethra, is an involuntary muscle controlling the flow of urine out of the bladder and into the urethra. The internal urethral sphincter also prevents the flow of semen into the bladder when males ejaculate and semen enters the prostatic urethra through the ejaculator ducts. The external urethral sphincter, located at the distal base of the prostate at the intersection between the prostatic urethra and the membranous urethra, is a voluntary muscle which controls the flow of urine from the prostate to the urethral meatus.

The prostate itself is anatomically composed of four lobes, the anterior lobe, the median lobe, the posterior lobe and the lateral lobes. The anterior, median and posterior lobes surround the urethra whereas the ejaculatory ducts pass through the median and posterior lobes. The lateral lobes are positioned around the anterior, median posterior lobes. An anatomical landmark on the prostatic urethra used in the classification of certain urethral development disorders is known as the verumontanum of the seminal colliculus. The verumontanum is located between the ejaculatory ducts, the prostatic utricle and the prostatic ducts, being inferior to the urethral crest.

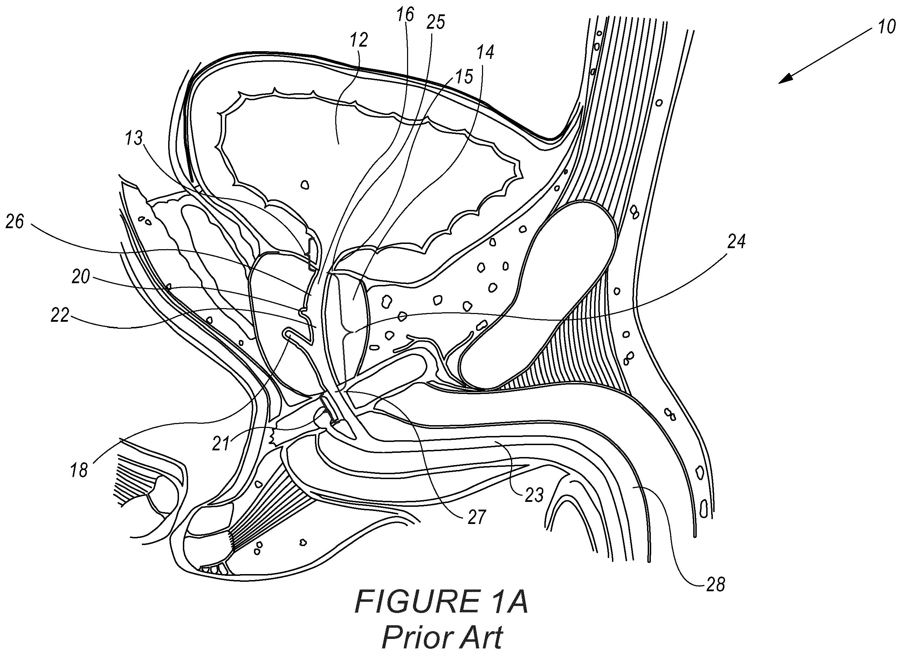

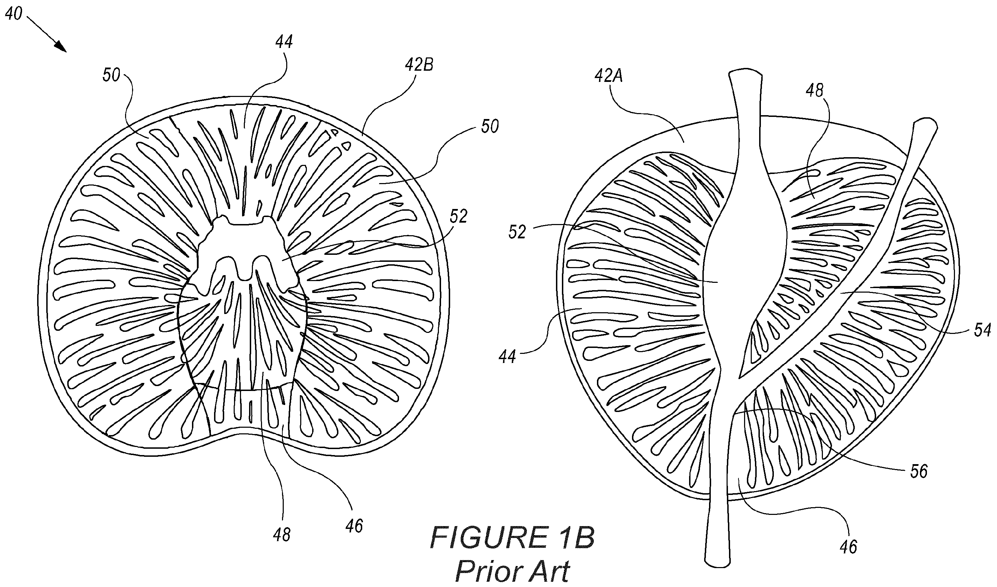

Reference is now made to Figures 1A and 1B, which are schematic illustrations respectively of the male reproductive system, the prostate and the urethra, generally referenced 10 and 40 respectively, as is known in the prior art. With reference to Figure 1A, shown is a schematic illustration of the male reproductive system from a sagittal view. As can be seen, a urinary bladder 12 connects with a urethra 16 which flows to a penis 28. A prostate 14, inferior to urinary bladder 12, surrounds urethra 16. The distal portion of urinary bladder 12 is shown as a bladder neck 15 which is proximate to prostate 14. The portion of urethra 16 which passes through bladder neck 15 is shown as a pre-prostatic urethra 13. The portion of urethra 16 which is surrounded by prostate 14 is shown as a prostatic urethra 24. Plurality of ejaculatory ducts 18 couple with urethra 16 in prostatic urethra 24. Shown within the section of prostatic urethra is a urethral crest 26, a prostatic utricle 20 and a verumontanum 22. The distal end of prostatic urethra 24 leads into a section of urethra 16 known as a membranous urethra 21 which then continues into a spongy urethra 23 which is surrounded by penis 28 and ends with a urethral meatus (not shown). As can also be seen, an internal urethral sphincter 25 is located at the intersection between pre-prostatic urethra 13 and prostatic urethra 24, and an external urethral sphincter 27 is located at the intersection between prostatic urethra 24 and membranous urethra 21.

With reference to Figure 18, shown is a schematic illustration of the prostate from a sagittal view 42A and a transverse view 428. Identical elements in sagittal view 42A and transverse view 428 are shown using the same reference numbers. As can be seen from sagittal view 42A, an anterior lobe 44, a posterior lobe 46 and a median lobe 48 surround a urethra 52. An ejaculatory duct 54 passes through posterior lobe 46 and median lobe 48. Shown as well is a verumontanum 56 on the proximal end of posterior lobe 46. As can be seen from transverse view 428, plurality of lateral lobes 50 surround anterior lobe 44, posterior lobe 46 and median lobe 48, with urethra 52 flowing between the various lobes of the prostate.

Common medical conditions of the prostate can include inflammation, non-cancerous enlargement of the prostate and prostate cancer. Non-cancerous enlargement of the prostate, also known as prostate enlargement occurs primarily in older men (in general +50) and is a medical condition in which the prostate grows in size but not due to metastasis or uncontrollable cell reproduction. As the prostate enlarges and grows in size, as can be seen from its anatomical position in human males such as shown in Figure 1A, it can apply pressure on the urethra, in particular the prostatic urethra, as well as adjacent anatomical sites such as the bladder neck (the inferior part of the bladder which connects with the urethra) and the ejaculatory ducts. In general, when the prostate enlarges, it is the lateral lobes which enlarge and place pressure on the prostatic urethra. However in some cases, the median lobe may also enlarge as well. Prostate enlargement can lead to a number of medical problems such as Benign Prostatic Hyperplasia (herein abbreviated **BPH),** prostatic Bladder Neck Obstruction (herein abbreviated **BNO)** and the like. BPH cause increased pressure on the prostatic urethra, making it difficult and painful to urinate. BNO can cause a complete obstruction of the prostatic urethra and an inability for the muscles around the bladder neck (for example the internal urethral sphincter) to relax and contract, making it nearly impossible to urinate and usually requiring medical intervention to relieve the bladder of urine.

Treatments for medical problems resulting from prostate enlargement range from medications taken orally (to reduce the size of the prostate by reducing hormone production), various kinds of stents and implants to enlarge the prostatic urethra, the use of catheter to enable a path from the bladder to the penis to expel urine as well as surgical procedures for removing either a portion of the prostate (such as transurethral resection of the prostate) or the entire prostate (such as a prostatectomy). Stents and implants for opening up the prostatic urethra are known in the art. PCT Patent Application Publication No. WO 2006/040767 A1 to Kilemnik and entitled "Prostate Treatment Stent" is directed to a tissue dissecting implant. The implant is spring-shaped and includes a plurality of rings elastically coupled there-between. Adjacent rings apply pressure on tissues caught between the rings, thereby pinching the caught tissues and inducing necrosis.

US Patent No. 8,715,239 B2 to Lamson et al., entitled "Devices, Systems, and Methods for Treating Benign Prostatic Hyperplasia and Other Conditions" is directed to a system including a rigid introducer device that is useable to facilitate insertion of an implant into the prostate gland. The implant includes a proximal anchor connected to a distal anchor by a tensioning element and the introducer device includes a rigid elongate body that is insertable into the subject's urethra. The introducer device also includes a rigid scope lumen configured to receive a cystoscope or other endoscopic device and a rigid working lumen. The working lumen is used to place a prostate compression implant and has an outlet through which the implant can be advanced through the wall of the urethra and to a position within or near the prostate gland.

US Patent No. 10,478,283 B2 to Bachar, entitled "Dilating Device for Prostatic Urethra" is directed to a device for dilating the prostatic urethra which comprises at least three, laterally connected ridges, wherein each ridge is configured to longitudinally engage with a different substantially longitudinal groove of the prostatic urethra of a patient. The laterally connected ridges are configured to laterally compress to enable insertion into the prostatic urethra in a compressed configuration, wherein the connected ridges are also configured to laterally expand to a normally-open configuration upon deployment within the prostatic urethra. Once deployed, the connected ridges exert a radially outwards force that dilates the prostatic urethra.

The prior art provides solutions to the pressure exerted on the prostatic urethra when the lateral lobes of the prostate enlarge, which is the more common form of BPH. However when the lateral lobes as well as the median lobe of the prostate enlarge, further complications may arise. As described below, an enlarged median lobe may exhibit sufficient movement within the prostatic urethra to obstruct the bladder neck after urination, thereby causing BNO. Regarding regular urination in males, as the muscles around the bladder contract and squeeze the bladder, the muscles around the bladder neck (such as the internal urethral sphincter) relax to let urine into the prostatic urethra. As urine is expelled from the bladder, air pressure is generated in the bladder which forces urine out of the bladder into the urethra. Once urination is finished and the muscles around the bladder relax and the muscles around the bladder neck contract to close the bladder neck, negative air pressure and suction may be generated in the prostatic urethra. In the case of BPH when only the lateral lobes of the prostate enlarge, the negative air pressure may have no effect on the opening of the bladder neck. However in the case of BPH when the lateral and median lobes enlarge, the negative air pressure (i.e.,suction) may pull the enlarged median lobe towards the bladder neck, thereby causing BNO as well. Whereas prior art solutions may provide relief to patients experiencing BPH by expanding and opening up the prostatic urethra, complications from BPH may further lead to BNO wherein the internal urethral sphincter becomes blocked. In such cases, merely expanding and opening up the prostatic urethra is insufficient to provide relief.

Prior are treatment methods in such cases (either BNO, BPH with BNO or BPH and BNO with an enlarged median lobe) usually use some form of laparoscopic surgery, such as transurethral resection of the prostate, or laser surgery, for removing a portion of the prostate, such as the median lobe, to ease symptoms. What is needed therefore is a minimally invasive treatment, such as an implant or stent device, capable of opening up the prostatic urethra to treat the symptoms of BPH while also keeping the internal urethral sphincter and the bladder neck both unobstructed, thereby also treating the symptoms of BNO.

### BRIEF DESCRIPTION OF THE DRAWINGS

The disclosed technique will be understood and appreciated more fully from the following detailed description taken in conjunction with the drawings in which:
Figures 1A and 1B are schematic illustrations of the male reproductive system and the prostate, as is known in the prior art;
Figures 2A, 2B and 2C are schematic illustrations of different embodiments of a first stent device in expanded form for expanding the inner walls of the prostatic urethra while also maintaining the bladder neck and internal urethral sphincter unobstructed, constructed and operative in accordance with an embodiment of the disclosed technique;
Figure 2D is a schematic illustration of the stent device of Figure 2A in a contracted form for insertion into the prostatic urethra, constructed and operative in accordance with another embodiment of the disclosed technique;
Figures 3A and 3B are schematic illustrations of different embodiments of a second stent device in expanded form for expanding the inner walls of the prostatic urethra while also maintaining the bladder neck and internal urethral sphincter unobstructed and for compressing the median lobe, constructed and operative in accordance with a further embodiment of the disclosed technique; and
Figure 4 is a schematic illustration showing the placement of a stent device within the prostatic urethra and bladder neck, constructed and operative in accordance with yet another embodiment of the disclosed technique.

### DETAILED DESCRIPTION OF THE EMBODIMENTS

The disclosed technique overcomes the disadvantages of the prior art by providing a stent device, such as a prostatic urethra implant, positioned within the prostatic urethra, wherein the shape of the stent device is elongated to include a prostatic urethra portion and a bladder neck portion. The stent includes at least one pressure wire which applies a radial force on the surrounding tissues of the inner wall of the prostatic urethra as well as the inner wall of the bladder neck. The pressure wire has a generally closed elliptical shape, similar to the shape of a wing. The pressure wire is made from a biocompatible metal which is elastic and has a shape memory such that it can be compressed for insertion into the urethra and then expanded once positioned in the prostatic urethra and the bladder neck. Once expanded, the pressure wire applies a radial force along the length of the prostatic urethra and the length of the bladder neck as well, thus enlarging the prostatic urethra and providing relief from BPH while also maintaining the internal urethral sphincter and the bladder neck unobstructed, thus also providing relief from BNO. The at least one pressure wire maintains an outwards radial pressure on the prostatic urethra as well as the internal urethral sphincter, thereby relieve constriction of the prostatic urethra while also keeping the bladder neck open. The stent, or implant, thus provides a solution to a constricted prostatic urethra due to Bi"H as well as to preventing BNO by maintaining the internal urethral sphincter unobstructed.

The stent device of the disclosed technique can be permanently positioned within the urethra of a patient or can be temporarily positioned therein and removed at a later time (i.e., the stent can be a retractable device). A cannula or sheath (for example as part of a cystoscope) inserted into the urethra via the urethral meatus can be used to positioned the stent or implant of the disclosed technique within the prostatic urethra. Surgical forceps can then be inserted into the cannula to precisely position the stent and hold it in position while the cannula is removed, thereby enabling the stent to open up into its expanded form. The surgical forceps can also be used to rotate the pressure wires of the stent to a desired position as well as moving the bladder neck portion of the pressure wires into the bladder neck of the patient. According to the disclosed technique, a reverse procedure can be used as well to collapse the stent and remove it from a patient by using forceps to compress the stent and pull it into the cannula for removal from the patient. As per the disclosed technique, the stent may be removed if outward radial pressure is no longer needed on the prostatic urethra, for example if the lobes of the prostate have reduced in size over time. Likewise, the stent may be kept permanently within the prostatic urethra to provide relief from BPH.

The stent device of the disclosed technique can be embodied have a single pressure wire or have a plurality of pressure wires. In one embodiment, the stent has two pressure wires, both shaped like wings, which are coupled together via a plurality of bridging elements. In a second embodiment, the stent has two pressure wires, also both shaped like wings, however which are coupled together directly without a plurality of bridging elements. In a third embodiment, the stent has a single pressure wire, shaped like a vase. In each of these embodiments, the stent device or implant of the disclosed technique includes a prostatic urethra portion as well as a bladder portion. In general, the shape of the pressure wires can also resemble the outline of a leaf or teardrop having a distal curvature for applying pressure radially outward. It is noted that the addition of a bladder portion to the stent device of the disclosed technique is one of the features which differentiates it from the prior art.

The stent can also include at least one additional wire for anchoring and holding in place an enlarged lobe of the prostate which may be contributing to BNO, for example, an enlarged median lobe. This additional wire can be referred to as an anchoring wire. The anchoring wire can be used as an anchor for simultaneously holding the stent device in place and preventing its migration into the bladder while also applying pressure to the median lobe of the prostate and thus preventing it from moving (due to suction) after urination. As described above, in some cases of BPH, not only do the lateral lobes of the prostate enlarge in prostate enlargement but the median lobe can enlarge as well. In such cases, the proximate side of the median lobe closest to the prostatic urethra can form a bulbous and dangling end. Due to the suction created when urinating, when a male ceases to urinate, the suction from the bladder may pull the bulbous and dangling end towards the bladder neck, thereby causing BNO. Thus the bulbous and dangling end of the median has sufficient movement to block the bladder neck even in cases where the prostatic urethra has been relieved of the general constriction caused by enlargement of the lateral lobes of the prostate. The at least one anchoring wire of the disclosed technique has a closed-shape and acts as an anchor for restricting movement of the bulbous and dangling end of an enlarged median lobe. Likewise, the anchoring wire is a further mechanism for preventing the stent device from migrating towards the bladder.

In another embodiment of the disclosed technique, the stent can have two anchoring wires, with each anchoring wire having a shape resembling the outline of a leaf, a leaflet or a teardrop. A first anchoring wire is used as an additional means to anchor the pressure wires of the stent such that the stent does not migrate to the bladder over time. A second anchoring wire is used to anchor the bulbous and dangling end of the median lobe such that it does not move when suction is generated after urination. The shape of both the first and second anchoring wires has a curvature which curves outwards, thereby being able to apply an outward radial pressure. The two anchoring wires are coupled at a proximal end of the stent and have different lengths corresponding to the structures of the prostatic urethra to which they are to anchor to. The first anchoring wire is long enough such that when the stent device is positioned within the prostatic urethra, the first anchoring wire places pressure of the verumontanum, thereby preventing the stent from migrating towards the bladder. The first anchoring wire can be used as an additional means for holding the stent in place without having to permanently affix the stent to tissues within the prostatic urethra (for example, without having to use a glue, sutures or other means for coupling implants with tissues in the body). The second anchoring wire is long enough such that when the stent is positioned within the prostatic urethra, the second anchoring wire places pressure of the bulbous and dangling end of the median lobe, thereby preventing the bulbous and dangling end of the median lobe from being sucked towards the bladder and obstructing the bladder neck after urination. Given the anatomical structure of the prostate and the prostatic urethra and that the stent (or implant) is inserted into the urethra from the penis, the second anchoring wire is longer than the first anchoring wire as the bulbous end of the median lobe is more distal to the penis than the verumontanum.

In the case of BPH which includes enlargement of the median lobe and the stent of the disclosed technique includes at least one anchoring wire, since the stent has an anchoring wire or extension for preventing the bulbous end of the median lobe from moving, the stent of the disclosed technique is meant to remain implanted within the prostatic urethra either permanently or semi-permanently. This is because the stent of the disclosed technique does not reduce the size of and/or change the composition of an enlarged median lobe, therefore the stent must remain permanently in place to prevent the bulbous end of the median lobe from causing BNO. In the event that the median lobe reduces in size (for example, through medication, through surgery or through other techniques) and the bulbous end of the median lobe is no longer a worry for causing BNO, then the stent of the disclosed technique can be removed from the prostatic urethra. In this respect, the stent of the disclosed technique can be semi-permanent.

In this description, the terms pressure and force (e.g., applying radial pressure or applying radial force) are employed interchangeably herein below, to describe the operation of the wires of the stent on the surrounding tissues and anatomical landmarks in the prostatic urethra and the bladder neck. That is, the wires (pressure and anchoring) are described as applying pressure on the tissues, or as applying an outward radial force on the tissues. Herein below, the terms proximal and distal refer to directions relative to the stent device. In particular, the distal end is the end of the stent device that is inserted into the body of the patient first and reaches the deepest (i.e., into the bladder neck). The proximal end is the end closer to the exit from the body of the patient and is located in prostatic urethra near the external urethral sphincter. Thus in reference to the disclosed technique, the bladder is the most distal point whereas the urethral meatus in the penis is the most proximal point.

The disclosed technique is in general described using the embodiment having two pressing wires having the worker skilled in the art can easily understand how the description can be modified to apply the disclosed technique to the other embodiments mentioned above. This applies to the embodiment having a single pressing wire as well as to the embodiments which further include one anchoring wire and two anchoring wires. The disclosed technique is described with reference to the human male reproductive system however the disclosed technique (i.e., the stent device) can equally be applied to the reproductive systems of male animals having a prostate gland or a gland which is anatomically and homologously similar to the human male prostate. In addition, the disclosed technique is described as a stent or stent device due to its similarity to the general function of a stent of keeping a passageway, in this case the prostatic urethra and the bladder neck, open and unconstricted. However the disclosed technique can also be referred to as an implant, such as a prostatic urethral implant and is at times described as such within the description. The terms "stent", "stent device" and "implant" in this respect are therefore interchangeable.

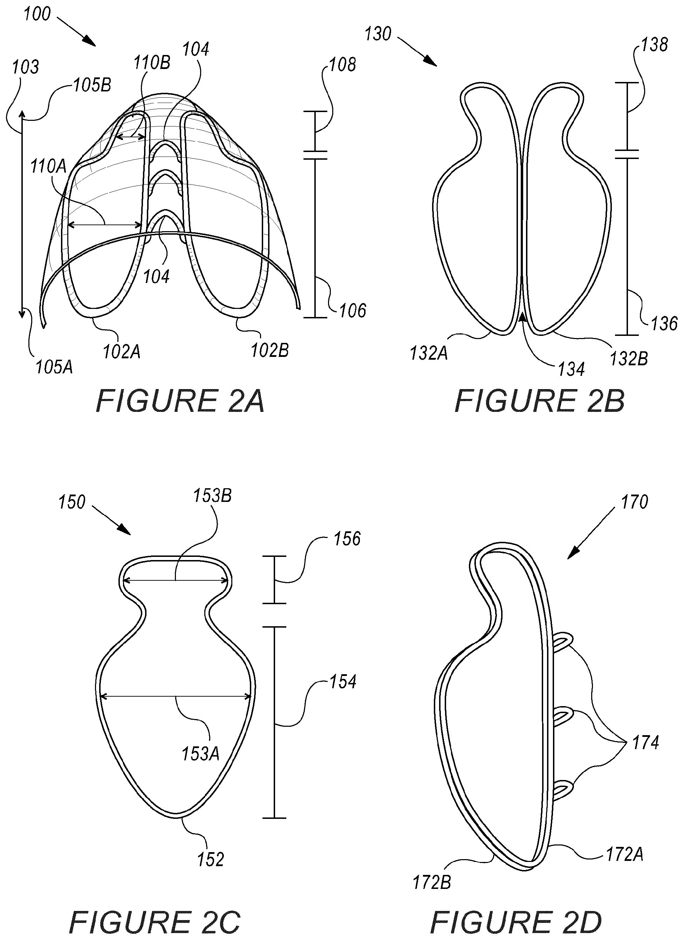

Reference is now made to Figures 2A, 2B and 2C, which are schematic illustrations of different embodiments of a first stent device in expanded form for expanding the inner walls of the prostatic urethra while also maintaining the bladder neck and internal urethral sphincter unobstructed, generally referenced 100, 130 and 150 respectively, constructed and operative in accordance with an embodiment of the disclosed technique. With specific reference to Figure 2A, shown is a first embodiment of a first stent device 100 in an expanded form. As shown, stent device 100 has two closed shaped sections 102A and 102B, both of which are shaped like wings and have a generally elliptical form. Closed shaped section 102A is substantially a mirror image of closed shaped section 102B. Each of closed shaped sections 102A and 102B has an outward radial curvature when in expanded form. Closed shaped sections 102A and 102B are coupled together via a plurality of bridging elements 104. Three bridging elements 104 are shown in Figure 2A, however any number of bridging elements are possible and are a design choice of stent device 100, from a single bridging element to many bridging elements. Closed shaped sections 102A and 102B and plurality of bridging elements 104 are each made from an elastic biocompatible metal that also has shape memory, such as nickel titanium (also known as nitinol), such that closed shaped sections 102A and 102B can be folded into a compressed form for insertion into the urethra for device placement. Each one of plurality of bridging element 104 has a generally curved shape in the form of an arch. All parts of stent device 100 should be made from biocompatible materials, such that there is no danger of infection to the body of the patient from stent device 100, which applies in general to all embodiments of the disclosed technique.

Stent device 100 is designed to be inserted into the urethra in the direction of an arrow 103, with the proximal end of stent device 100 being shown by reference 105A and with the distal end of stent device 100 being shown by reference 105B. Shown as well is the division of each of closed shaped sections 102A and 102B into a prostatic urethra section 106 and a bladder neck section 108. Even though each of closed shaped sections 102A and 102B are closed shapes, the width of each closed section varies along the length of stent device 100, in the direction of arrow 103. As shown, in prostatic urethra section 106, closed shaped section 102A has a width shown by an arrow 110A whereas in bladder neck section 108, closed shaped section 102A has a width shown by an arrow 110B. As can be seen arrow 110B is significantly shorted than arrow 110A.

Stent device 100 is designed to be positioned within the prostatic urethra of a patient (not shown) such that each of closed shaped sections 102A and 102B applies outward radial pressure on the prostatic urethra in order to expand it and enlarge it and apply outward pressure on enlarged lobes that may be constricting the prostatic urethra and causing BPH. The width of prostatic urethra section 106 shown as arrow 110A is wide enough to apply sufficient pressure on the inner walls of the prostatic urethra to relieve it of constriction due enlarged lobes of the prostate. The distal portions of closed shaped sections 102A and 102B likewise apply outward pressure on the bladder neck to prevent it from obstruction. The width of bladder neck section 108 shown as arrow 110B is wide enough to apply sufficient pressure on the inner walls of the pre-prostatic urethra to prevent bladder neck obstruction. Stent device 100 is positioned within the urethra such that prostatic urethra section 106 is positioned within the prostatic urethra of a patient and bladder neck section 108 is positioned within the bladder neck of the patient, thus substantially in the pre-prostatic urethra of the patient. As shown, closed shaped sections 102A and 102B have sufficient length in the direction of arrow 103 such that proximal end 105A of stent device 100 is located at the proximal end of the prostatic urethra near the external urethral sphincter (not shown) whereas distal end 105B of stent device 100 is located within the bladder neck near the internal urethral sphincter (not shown). It is noted that the difference in width between prostatic urethra section 106 and bladder neck section 108 (meaning the difference in length between arrow 110A and arrow 110B) ensures that stent device 100 does not migrate into the bladder over time. As mentioned above, after urination a negative pressure can be built up in the bladder which can pull anatomical elements in the prostatic urethra towards the bladder (which is discussed below). The negative pressure can also theoretically pull a stent device placed in the prostatic urethra towards the bladder, thus causing further medical complications by the movement of the stent device out of its intended position within the urethra. According to the disclosed technique, the bladder neck section of the stent device has a significantly smaller width that the prostatic urethra section in order to simultaneously enable outward radial pressure to be applied to the bladder neck while also preventing the stent device from migrating towards the bladder and substantially remaining in its implanted positioned without having to use additional and/or external elements to keep the stent device properly positioned. As mentioned above, stent device 100 can be implanted permanently, semi-permanently and temporarily in a patient, depending on the patient's condition. Unlike prior art devices which may only offer relief to BPH by expanding the prostatic urethra, disclosed technique, such as it is embodied in stent device 100, can provide relief to BPH as well as to BNO due to the increased length of each one of closed shaped sections 102A and 102B.

With specific reference to Figure 2B, shown is a second embodiment of the first stent device 130 in an expanded form. Similar to stent device 100 (Figure 2A), stent device 130 includes two closed shaped sections 132A and 132B, both of which are shaped like wings and have a generally elliptical form. Closed shaped section 132A is substantially a mirror image of closed shaped section 132B. Each of closed shaped sections 132A and 132B has an outward radial curvature when in expanded form. And similar to stent device 100, stent device 130 has a prostatic urethra section 136 and a bladder neck section 138, wherein prostatic urethra section 136 is positioned within the prostatic urethra of a patient and bladder neck section 138 is positioned within the bladder neck of the patient. As shown, stent device 130 does not include any bridging elements. Closed shaped section 132A is thus coupled directly with closed shaped section 132B, shown by an arrow 134. The coupling of the two closed shaped sections shown by arrow 134 can be via being glue, a weld joint, a pin, a hinge and the like.

In general, stent device 130 is substantially similar to stent device 100 (Figure 2A) in terms of materials of construction and flexibility and functions in a similar manner to keep the prostatic urethra unconstricted while also keeping the bladder neck unobstructed. And similar to stent device 100, the difference in width (not illustrated) between prostatic urethra section 136 and bladder neck section 138 ensures that stent device 130 does not migrate into the bladder neck and the bladder of the patient but rather remains positioned between the external urethral sphincter (at the proximal end of the prostatic urethra) and the internal urethral sphincter (at the distal end of the bladder neck) without any external elements (such as supports, sutures and the like).

With specific reference to Figure 2C, shown is a third embodiment of the first stent device 150 in an expanded form. Unlike stent devices 100 (Figure 2A) and 130 (Figure 2B), stent device 150 includes only a single closed shaped section 152, generally shaped like a vase. It will be seen that the vase-like shape of stent device 150 resembles the double wing shape of stent devices 100 and 130 and includes both a prostatic urethra section 154 and a bladder neck section 156.

As shown, a maximum width 153A of prostatic urethra section 154 is significantly larger than a maximum width 153B of bladder neck section 156. When positioned properly within a patient, prostatic urethra section 154 is positioned within the prostatic urethra of the patient and bladder neck section 156 is positioned within the bladder neck of the patient.

Closed shaped section 152 has an outward radial curvature when in expanded form and is made from a biocompatible, flexible metal having shape memory. Stent device 150 does not include any bridging elements and consists of a single closed shaped section, thus being cost effective to manufacture. Similar to stent devices 100 (Figure 2A) and 130 (Figure 2B), stent device 150 functions in a similar manner to keep the prostatic urethra unconstricted while also keeping the bladder neck unobstructed. And similar to stent devices 100 and 130, the difference in widths 153A and 153B between prostatic urethra section 154 and bladder neck section 156 ensures that stent device 150 does not migrate into the bladder neck and the bladder of the patient but rather remains positioned between the external urethral sphincter (at the proximal end of the prostatic urethra) and the internal urethral sphincter (at the distal end of the bladder neck) without any external elements (such as supports, sutures and the like).

Reference is now made to Figure 2D, which is a schematic illustration of the stent device of Figure 2A in a contracted form for insertion into the prostatic urethra, generally referenced 170, constructed and operative in accordance with another embodiment of the disclosed technique. As shown, stent device 170 is substantially similar to stent device 100 (Figure 2A), including two closed shaped sections 172A and 172B as well as a plurality of bridging elements 174. In Figure 2D, closed shaped sections 172A and 172B have been folded over one another and plurality of bridging element 174 are folded in half. Due to the outward radial pressure which closed shaped sections 172A and 172B can exert when in their expanded configuration, in their contracted form as shown in Figure 2D, closed shaped sections 172A and 172B must be kept in a sheath, cannula and/or delivery tube to remain in their contracted form.

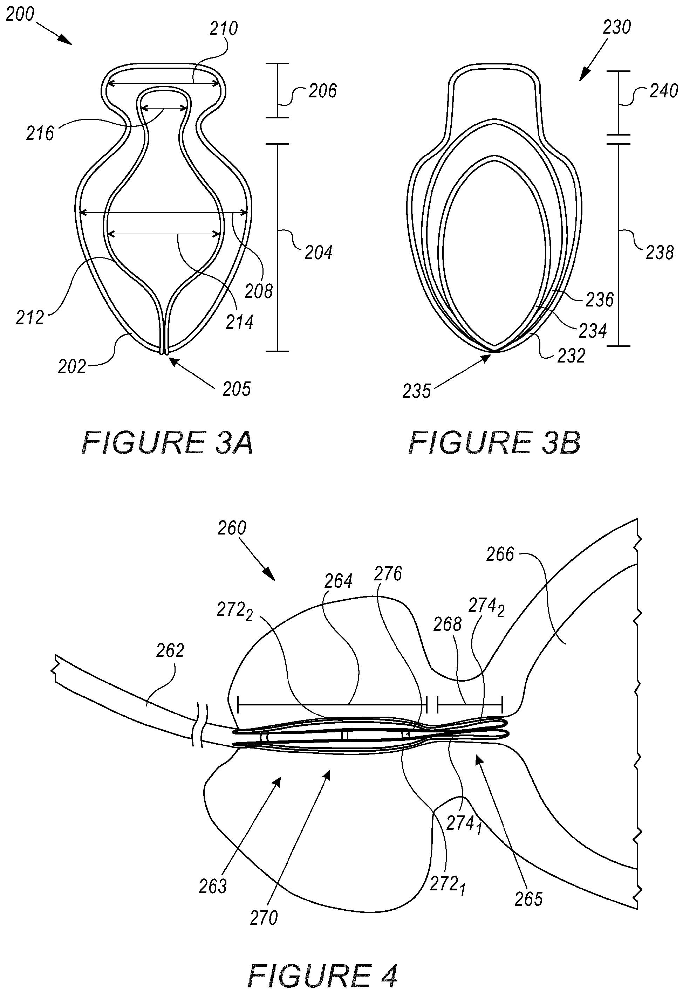

Once the sheath, cannula and/or delivery tube is inserted into the urethra for placement of the stent device, the sheath, cannula and/or delivery tube is removed while holding the stent device in place. As the sheath, cannula and/or delivery tube is removed, closed shaped sections 172A and 172B will open up into their expanded configuration as shown in Figure 2A (and as shown below in Figure 4). Stent devices 130 (Figure 28) and 150 (Figure 2C) would have similar shapes when folded into their contracted form.

Reference is now made to Figures 3A and 3B, which are schematic illustrations of different embodiments of a second stent device in expanded form for expanding the inner walls of the prostatic urethra while also maintaining the bladder neck and internal urethral sphincter unobstructed and for compressing the median lobe, generally referenced 200 and 230 respectively, constructed and operative in accordance with a further embodiment of the disclosed technique. With specific reference to Figure 3A, stent device 200 as shown is substantially similar to stent device 150 (Figure 2C) and includes a single closed shaped section 202, have a vase-like shape and including a prostatic urethra section 204 and a bladder neck section 206. As shown, the maximum width of prostatic urethra section 204 is shown via an arrow 208 and the maximum width of bladder neck section 206 is shown via an arrow 210.

Stent device 200 differs from stent device 150 (Figure 2C) in that stent device 200 also includes an anchoring wire 212, coupled with closed shaped section 202 at its proximal end, shown as a base section 205. Anchoring wire 212 has a similar vase-like shape to closed shaped section 202, with a maximum width in prostatic urethra section 204 being shown by an arrow 214 and a maximum width in bladder neck section 206 being shown by an arrow 216. Anchoring wire 212, similar to closed shaped section 202, is made from a biocompatible flexible metal having shape memory. In general, both closed shaped section 202 and anchoring wire 212 apply outward radial pressures with anchoring wire 212 having an outward radial curvature. Closed shaped section 202 is used to keep the prostatic urethra unconstricted while also maintaining the bladder neck unobstructed. Anchoring wire 212 is used to put pressure on the median lobe (not shown) of a patient by compressing it. Anchoring wire 212 thus keeps an enlarged median lobe from moving around and migrating towards the bladder neck, which might occur if the enlarged median lobe has a bulbous end that can be pulled towards the bladder neck after urination due to negative pressure. In general, it is the distal end of anchoring wire 212 that puts pressure on the bulbous end of the enlarged median lobe thus ensuring that it does not move and/or migrate towards the bladder once positioned in the urethra.

Base section 205 may include a cap (not shown) which can be used to rotate stent device 200 when placed within the urethra. The cap has a hollow space which allows urine to pass there through as the cap is placed within the urethra. The hollow space can also be used by a physician or surgeon for rotating stent device 200 and orienting anchoring wire 212 appropriately such that it compresses the median lobe. In general, the hollow space does not have a round shape, thus enabling the cap to rotate with the aid of a tool (not shown) having a complementary shape to the hollow space. The non-round hollow space of the cap is configured to receive a corresponding non-round pin (i.e., a tool) and to transfer rotational motion of the pin to stent device 200.

With specific reference to Figure 3B, stent device 230 as shown is substantially similar to stent device 200 (Figure 3A) and includes a single closed shaped section 232, have a vase-like shape and including a prostatic urethra section 238 and a bladder neck section 240. Stent device 230 differs from stent device 200 in that stent device 230 includes a first anchoring wire 234 and a second anchoring wire 236, both coupled with closed shaped section 232 at its proximal end, shown as a base section 235. First anchoring wire 234 and second anchoring wire 236 each has a leaf-like shape, and similar to closed shaped section 232, are made from a biocompatible flexible metal having shape memory. In general, closed shaped section 232, first anchoring wire 234 and second anchoring wire 236 apply outward radial pressures, with first and second anchoring wires 234 and 236 having outward radial curvatures. Closed shaped section 232 is used to keep the prostatic urethra unconstricted while also maintaining the bladder neck unobstructed. First anchoring wire 234 can be used as an additional anchor for putting pressure on the verumontanum of the prostatic urethra, thus further ensuring that stent device 230 does not move while positioned inside the urethra. In general, the distal end of first anchoring wire 234 is positioned distal to the verumontanum. Second anchoring wire 236 is similar to anchoring wire 212 (Figure 3A) and its distal end is used to put pressure on the median lobe (not shown) of a patient by compressing it. Second anchoring wire 236 thus keeps an enlarged median lobe from moving around and migrating towards the bladder neck, which might occur if the enlarged median lobe has a bulbous end that can be pulled towards the bladder neck after urination due to negative pressure (i.e., suction).

Similar to stent device 200 (Figure 3A), base section 235 may include a cap (not shown) which can be used to rotate stent device 230 when placed within the urethra. The cap has a hollow space which allows urine to pass there through as the cap is placed within the urethra. The hollow space, which has a non-round shape as described above in Figure 3A, can also be used by a surgeon for rotating stent device 230 and orienting first anchoring wire 234 appropriately such that it compresses the verumontanum of the prostatic urethra and orienting second anchoring wire 236 appropriately such that it compresses the median lobe. Similar to the first stent device described above in Figures 2A-2C, stent devices 200 and 230 can be implanted permanently, semi-permanently and temporarily, depending on the condition of the patient.

In general, after any of the stent devices of the disclosed technique are implanted, a patient can resume his regular lifestyle without any hindrances. For example, the pressure applied by first anchoring wire
234 and second anchoring wire 236 is sufficient for maintaining the position of stent device 230 in the prostatic urethra and for compressing the median lobe without causing any pain and/or discomfort to the patient.

In addition, the shape of first and second anchoring wires 234 and 236 is unobtrusive (as the shapes of the anchoring wires are substantially hollow), thereby enabling fluids and liquids (such as urine, prostatic secretions, semen and the like) to pass over and through first and second anchoring wires 234 and 236 without hindrance. In general, the length of first and second anchoring wires 234 and 236 (as well as anchoring wire 212 (Figure 3A)) may need to be altered accordingly to serve their respective functions of compressing and anchoring. The pressure applied by closed shaped section 232 on the prostatic urethra and the bladder neck is sufficient for opening up the prostatic urethra and the bladder neck and easily enabling urine to pass there through without discomfort. It is noted as well that any of the stent devices of the disclosed technique described herein can be implanted in any tubular organ that requires relief of a constriction while simultaneously restricting the mobility of anatomical structures, such as tubular organs of the digestion system, blood vessels and the like.

As shown in Figure 3A, second stent device 200 can provide relief to constriction of the urethra, can maintain the bladder neck open and can apply pressure to the bulbous end of the median lobe for preventing BNO. As shown in Figure 3B, second stent device 230 can provide relief to constriction of the urethra, can maintain the bladder neck open, can apply pressure to the bulbous end of the median lobe for preventing BNO and can also compress the verumontanum for providing an additional anchor for maintaining the position of stent device 230 when placed in the urethra.

It is noted that the closed shaped sections of stent devices 100, 130, 150, 200 and 230 (in Figures 2A-2C and 3A-3B) can be made from flexible biocompatible metal wire having a cross-section which is as thin as 0.5 millimeters. Such a thickness of metal wire should be strong enough to apply a force on the surrounding tissues of the prostate to relieve constriction of the prostatic urethra and to maintain the bladder neck open. Likewise, the anchoring wires in stent device 200 and 230 should be strong enough to apply enough of a radial force to compress the median lobe and the verumontanum such that the passage of fluids and liquids through the urethra does not dislodge the anchoring wires. Thus the anchoring wires can also have a cross-section which is as thin as 0.5 millimeters.

Reference is now made to Figure 4, which is a schematic illustration showing the placement of a stent device within the prostatic urethra and bladder neck, generally referenced 260, constructed and operative in accordance with yet another embodiment of the disclosed technique. Figure 4 shows a sagittal plane cross-section of the prostatic urethra and bladder neck with a stent device 270 (substantially similar for example with stent device 100 of Figure 2A) of the disclosed technique positioned therein. A urethra 262 is shown, along with a prostatic urethra 263, a bladder neck 265 and a bladder 266. Stent device 270 is shown having a prostatic urethra section 264 and a bladder neck section 268. Stent device 270 includes two closed shaped sections 272₁ and 272₂ which are coupled together via a plurality of bridging elements 276. The distal ends of closed shaped sections 272₁ and 272₂ are shown as distal ends 274₁ and 274₂ in bladder neck section 268.

Once implanted, as shown, the proximal end of stent device 270 sits at the proximal end of the prostatic urethra, near the external urethral sphincter while the distal end of stent device 270 sits at the distal end of the bladder neck, near the internal urethral sphincter. The portion of closed shaped sections 272₁ and 272₂ within the prostatic urethra apply an outward radial force along the tissues of the inner wall of the prostatic urethra, thus opening up prostatic urethra 263 and relieving it of constriction in the case of BPH. Distal ends 274₁ and 274₂ maintain bladder neck 265 open, thus keeping it unobstructed in the case of BNO. As mentioned above, stent device 270 is either permanently, semi-permanently or temporarily placed within the prostatic urethra and the bladder neck.

It will be appreciated by persons skilled in the art that the disclosed technique is not limited to what has been particularly shown and described hereinabove. Rather the scope of the disclosed technique is defined only by the claims, which follow.

## Claims

1. Stent device for enlarging the prostatic urethra and for unobstructing the bladder neck of a patient, said stent device comprising: two closed-shaped pressure wires, each of said pressure wires having a prostatic urethra section and a bladder neck section, for exerting an outward radial pressure on said prostatic urethra and said bladder neck; and a plurality of bridging elements, for coupling said two closed-shaped pressure wires together along a length of said pressure wires, wherein each of said closed-shaped pressure wires has a generally closed elliptical shape; wherein a width of said prostatic urethra section is larger than a width of said bladder neck section of each of said pressure wires; wherein each of said two pressure wires and said plurality of bridging elements are elastic and have shape memory thereby giving said stent device an expanded configuration and also being compressible into a compressed configuration; and wherein in said expanded configuration, said pressure wires exert said outward radial pressure on said prostatic urethra and said bladder neck.

2. The stent device according to claim 1, wherein said two closed-shaped pressure wires and said plurality of bridging elements are made from a material selected from the list consisting of: nickel titanium (nitinol); and a biocompatible metal.

3. The stent device according to claim 1, wherein said generally closed elliptical shape resembles a shape of a wing.

4. Stent device for enlarging the prostatic urethra and for unobstructing the bladder neck of a patient, said stent device comprising: two closed-shaped pressure wires, each of said pressure wires having a prostatic urethra section and a bladder neck section, for exerting an outward radial pressure on said prostatic urethra and said bladder neck, wherein each of said closed-shaped pressure wires has a generally closed elliptical shape; wherein said two closed-shaped pressure wires are coupled together via a coupling along a length of said pressure wires; wherein a width of said prostatic urethra section is larger than a width of said bladder neck section of each of said pressure wires; wherein each of said two pressure wires are elastic and have shape memory thereby giving said stent device an expanded configuration and also being compressible into a compressed configuration; and wherein in said expanded configuration, said pressure wires exert said outward radial pressure on said prostatic urethra and said bladder neck.

5. The stent device according to claim 4, wherein said two closed-shaped pressure wires are made from a material selected from the list consisting of:
nickel titanium (nitinol); and
a biocompatible metal,
and/or
wherein said generally closed elliptical shape resembles a shape of a wing,
and/or
wherein said coupling is selected from the list consisting of:
being glued;
a weld joint;
a pin; and
a hinge.

6. Stent device for enlarging the prostatic urethra and for unobstructing the bladder neck of a patient, said stent device comprising: a closed-shaped pressure wire, having a prostatic urethra section and a bladder neck section, for exerting an outward radial pressure on said prostatic urethra and said bladder neck, wherein said closed-shaped pressure wire has a generally closed elliptical shape; wherein a width of said prostatic urethra section is larger than a width of said bladder neck section of each of said pressure wires; wherein said pressure wire is elastic and has shape memory thereby giving said stent device an expanded configuration and also being compressible into a compressed configuration; and wherein in said expanded configuration, said pressure wire exerts said outward radial pressure on said prostatic urethra and said bladder neck.

7. The stent device according to claim 6, wherein said closed-shaped pressure wire is made from a material selected from the list consisting of:
nickel titanium (nitinol); and
a biocompatible metal
and/or
wherein said generally closed elliptical shape resembles a shape of a vase.
and/orfurther comprising an anchoring wire, coupled with a proximal end of said pressure wire, having a closed shaped.

8. The stent device according to claim 6, further comprising an anchoring wire, coupled with a proximal end of said pressure wire, having a closed shaped
and/or
wherein said anchoring wire has a width which is shorter than said width of said prostatic urethra section
and/or
wherein said anchoring wire is for compressing a median lobe..

9. The stent device according to claim 6, further comprising two anchoring wires, each coupled with a proximal end of said pressure wire, having closed shapes.

10. The stent device according to claim 9, wherein said two anchoring wires each have a width which is shorter than said width of said prostatic urethra section.

11. The stent device according to claim 9, wherein a first one of said two anchoring wires is for compressing a median lobe and wherein a second one of said two anchoring wires is for anchoring said stent device in said prostatic urethra.

12. The stent device according to claim 9, wherein a first one of said two anchoring wires is longer than a second one of said two anchoring wires and wherein said two anchoring wires are shorter in length that said closed-shaped pressure wire.

13. The stent device according to claim 8, further comprising a cap, coupled with said proximal end of said pressure wire, for coupling said anchoring wire with said pressure wire, said cap comprising a hollow space.

14. The stent device according to claim 9, further comprising a cap, coupled with said proximal end of said pressure wire, for coupling said two anchoring wires with said pressure wire, said cap comprising a hollow space.

15. The stent device according to one of claims 13 and 14, wherein said hollow space of said cap comprises a non-round shape.

16. The stent device according to claim 15, wherein said hollow space is configured to accept a corresponding mechanism; and wherein said non-round shape is configured to transfer rotational motion of said corresponding mechanism to said stent device,
and/or
wherein said corresponding mechanism is selected from the list consisting of:
a pin; and
a tool.
